(19) Europäisches Patentamt
European Patent Office
Office européen des brevets

(11) **EP 2 062 883 B1**

(12) **EUROPEAN PATENT SPECIFICATION**

(45) Date of publication and mention
of the grant of the patent:
**11.04.2012 Bulletin 2012/15**

(21) Application number: **07805811.2**

(22) Date of filing: **05.09.2007**

(51) Int Cl.:
*A61K 31/353* (2006.01)     *A61K 36/18* (2006.01)
*A61P 3/04* (2006.01)     *A61P 3/10* (2006.01)
*A61P 5/50* (2006.01)     *A23L 1/30* (2006.01)

(86) International application number:
**PCT/JP2007/000956**

(87) International publication number:
**WO 2008/029511 (13.03.2008 Gazette 2008/11)**

(54) **CATECHIN AS INSULIN SENSITIVITY IMPROVING AGENT**

CATHECHINE ALS MITTEL ZUM ERHÖHEN DER INSULINSENSIBILITÄT

CATECHINE COMME AGENT AMÉLIORANT LA SENSIBILITÉ À L'INSULINE

(84) Designated Contracting States:
**DE FR GB**

(30) Priority: **08.09.2006 JP 2006244772**
**18.10.2006 JP 2006283449**

(43) Date of publication of application:
**27.05.2009 Bulletin 2009/22**

(73) Proprietor: **Kao Corporation**
**Chuo-ku**
**Tokyo 103-8210 (JP)**

(72) Inventor: **NAGAO, Tomonori**
**Tokyo 131-8501 (JP)**

(74) Representative: **HOFFMANN EITLE**
**Patent- und Rechtsanwälte**
**Arabellastraße 4**
**81925 München (DE)**

(56) References cited:
**JP-A- 2002 080 362     JP-A- 2003 081 825**

• FUKINO YOKO ET AL: "Randomized controlled trial for an effect of green tea consumption on insulin resistance and inflammation markers.", JOURNAL OF NUTRITIONAL SCIENCE AND VITAMINOLOGY OCT 2005 LNKD- PUBMED: 16392704, vol. 51, no. 5, October 2005 (2005-10), pages 335-342, XP9142236, ISSN: 0301-4800

• RYU O H ET AL: "Effects of green tea consumption on inflammation, insulin resistance and pulse wave velocity in type 2 diabetes patients", DIABETES RESEARCH AND CLINICAL PRACTICE, AMSTERDAM, NL, vol. 71, no. 3, 1 March 2006 (2006-03-01), pages 356-358, XP025071355, ISSN: 0168-8227, DOI: DOI: 10.1016/J.DIABRES.2005.08.001 [retrieved on 2006-03-01]

• KAO YUNG-HSI ET AL: "Tea, obesity, and diabetes", MOLECULAR NUTRITION & FOOD RESEARCH, WILEY - VCH VERLAG, WEINHEIM, DE, vol. 50, no. 2, 1 February 2006 (2006-02-01), pages 188-210, XP002462206, ISSN: 1613-4125, DOI: DOI:10.1002/MNFR.200500109

• THIELECKE F ET AL: "The potential role of green tea catechins in the prevention of the metabolic syndrome - A review", PHYTOCHEMISTRY, PERGAMON PRESS, GB, vol. 70, no. 1, 1 January 2009 (2009-01-01), pages 11-24, XP025942110, ISSN: 0031-9422, DOI: DOI: 10.1016/J.PHYTOCHEM.2008.11.011 [retrieved on 2009-01-13]

• MATSUYAMA TAKESHI ET AL: "Catechin safely improved higher levels of fatness, blood pressure, and cholesterol in children.", OBESITY (SILVER SPRING, MD.) JUN 2008 LNKD- PUBMED:18356827, vol. 16, no. 6, June 2008 (2008-06), pages 1338-1348, XP002613462, ISSN: 1930-7381

• LI R.W. ET AL.: 'Green tea leaf extract improves lipid and glucose homeostasis in a fructose-fed insulin-resistance hamster model' JOURNAL OF ETHNOPHARMACOLOGY vol. 104, no. 1-2, March 2006, pages 24 - 31, XP022306856

EP 2 062 883 B1

- LIU H-S. ET AL.: 'Inhibitory effect of green tea (-)-epigallocatechin gallate on resistin gene expression in 3T3-L1 adipocytes depends on the ERK pathway' AMERICAN JOURNAL OF PHYSIOLOGY - ENDOCRINOLOGY AND METABOLISM vol. 290, no. 2, February 2006, pages E273 - E281, XP003021659

- CABRERA C. ET AL.: 'Beneficial effects of green tea - A review' JOURNAL OF THE AMERICAN COLLEGE OF NUTRITION vol. 25, no. 2, April 2006, pages 79 - 99, XP003021660

**Description**

Field of the Invention

The present invention relates to an insulin sensitivity-improving agent for obese children according to claim 1.

Background of the Invention

In recent years, the number of diabetic patients is on the rise and comes to be one of the serious social problems in Japan. Type 2 diabetes, accounting for more than 90% of diabetes, is characterized by the conditions under which the insulin secretion activity is sharply weakened by the varying correlation between the decrease in the insulin secretion from pancreatic beta cells and the decrease in the insulin sensitivity in insulin target organs (i.e. skeletal muscles, the liver and adipose tissues), thereby resulting in hyperglycemia. There is a strong possibility that the decrease in insulin secretion could be affected primarily by the so-called genetic factor, and it is also suggested that the insulin sensitivity might be associated largely with the obesity attributed to environmental factors such as overeating, high fat diets, and lack of exercise, in conjunction with genetic predisposition. An obese person is found to carry hypertrophic fat cells, and these fat cells produce large amounts of cytokines such as tumor necrosis factor (TNFα) and resistin and free fatty acids. These TNFα, resistin, and free fatty acids cause a decrease in insulin sensitivity by interrupting the insulin signal transduction in the skeletal muscles and the liver. As a result, the excessive secretion of insulin from an organism takes place to control a blood sugar level. However, the number of insulin receptors on the target organs of insulin is decreased due to hyperinsulinemia, and exhausted pancreatic beta cells undermine the insulin secreting ability, leading to a diabetic condition. As long as a hyperglycemic condition persists, glucose itself enhances the decreases in insulin secretion from pancreatic beta cells and the insulin sensitivity in the periphery, and gives rise to glucose toxicity. Thus a vicious cycle is created, and the disorder further worsens. Therefore, a drug for improving insulin sensitivity is thought to be greatly useful as a therapeutic agent for diabetes (Non-patent Documents 1 to 4).

From such a viewpoint, insulin sensitivity improving drugs were developed previously, and some of such drags are commercially available today. However, thiazolidine drugs, known as insulin sensitivity improving drugs, have a problem with severe hepatopathy.

Furthermore, the number of pediatric patients with type 1 diabetes is conventionally known to be more than that of patients with type 2 diabetes, but it has recently been pointed out that the number of type 2 diabetic patients is sharply rising along with the increase in the obese population. Unlike type 1 diabetes, type 2 diabetes normally does not cause any subjective symptom, so that no drug therapy is necessary in most of the cases. Thus it is reported statistically that the type 2 diabetic patients are rather more susceptible to contracting complicated symptoms such as retinopathy, nephropathy, neuropathy and arteriosclerosis, than type 1 diabetic patients whose blood sugar levels tend to increase more easily. Childhood diabetes is affected largely by genetic factors and is often associated with obesity. Furthermore, the onset of childhood diabetes occurs at the earlier age of its patient, so the complicated symptoms associated therewith could lead to severe disorders while they are still young.

A decrease in insulin sensitivity is often recognized as one of the major causes for childhood obesity. If a therapeutic agent for adult diabetic patients is administered to such obese children, there is a high possibility that some adverse effect (e.g. severe hepatopathy typical of adult) could be produced more strongly by such a therapy. Thus it is the present situation that a drug for improving insulin sensitivity in obese children, which can be used safely, has yet to exist.

Furthermore, catechins contained in green teas, black teas, oolong teas and the like are acknowledged as having physiological benefits in suppression of increases in cholesterol levels (Patent Document 1), α-amylase activity inhibition (Patent Document 2), and the like. However, their effects on insulin sensitivity in obese children are unknown.

Patent Document 1: JP-A-60-156614

Patent Document 2: JP-A-3-133928

Non-patent Document 1: Biochemistry, Vol. 71, No. 11, pp. 1281-1298, 1999

Non-patent Document 2: Chemistry and Biology, Vol. 37, No. 2, pp. 120-125

Non-patent Document 3: Laboratory Tests, Vol. 42, No. 4, pp. 395-403, 1998

Non-patent Document 4: Mebio Separate Edition: Multiple risk factor syndrome 2, 1999

Fukino Yoko et al., J. Nutrit. Sci. Vit. 51(5), Oct. 2005, p. 335-342 concerns a study relating to the effect of catechin rich drinks on the insulin resistance in adults with borderline diabetes or diabetes. The test persons had a body mass index (BMI) of around $25.5 \pm 4.8$ (intervention group) and $25.9 \pm 3.9$ (control group).

In Ryu O H et al., Diabetes Res. Clin. Pract. 71(3), 1 March 2006, p. 356-358, the effect of green tea consumption on insulin resistance in type 2 diabetes patients having a BMI of $25.0 \pm 2.2$ is examined.

Disclosure of the Invention

The present invention provides a catechin for use in a method for improving insulin sensitivity in an obese child with a quantitative insulin sensitivity check index (QUICKI) of 0.31 or lower.

Brief Description of the Drawings

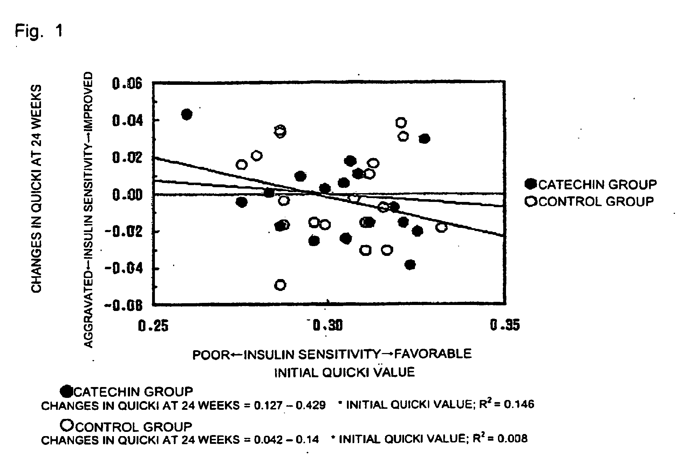

Figure 1 shows changes in the quantitative insulin sensitivity check index (QUICKI) after taking a catechin for 24 weeks.

Detailed Description of the Invention

The present invention provides an insulin sensitivity-improving agent for obese children with a QUICKI of 0.31 or lower, which has a reduced effect on changes in energy intake and can be used by an easily self-manageable administration method for a long period of time because its use is targeted at growing children.

Accordingly, the present inventors examined effects of catechins on insulin sensitivity in obese children. As a result, it has been found that the insulin sensitivity in obese children is markedly improved by the continued intake of catechins.

When the insulin sensitivity-improving agent of the present invention is used, insulin sensitivity can be markedly improved in obese children. The long-period intake of this agent is possible, moreover.

Catechins used in the present invention are preferably non-polymer catechins. Specifically, "non-polymer catechins" is a generic term encompassing all of non-epi-non-polymer catechins (A), such as catechin, gallocatechin, catechin gallate, and gallocatechin gallate and epi-non-polymer catechins (B), such as epicatechin, epigallocatechin, epicatechin gallate, and epigallocatechin gallate combined.

Catechins used in the present invention are extracted with water or hot water from tea leaves of green teas such as sencha, gyokuro, and sweet tea, semi-fermented teas such as Ti Kuan Yin, Shikishu, huang jin gui, and Wu Yi Rock tea, which are collectively called oolong tea, fermented teas called black tea, which are produced in Darjeeling, Assam, Sri Lanka, and the like, obtained by processing tea leaves of the genus Camellia, such as, for example, *C. sinensis* and *C. assamica,* Yabukita species, or hybrids of these species.

The extraction of tea is carried out by conventional methods such as stirring extraction. Organic acids or organic acid salts, such as sodium ascorbate, may be added to water at the time of extraction. A tea extract may also be obtained in combination with a method of extraction under the so-called non-oxidative atmosphere while removing dissolved oxygen by deaeration by boiling, or by aeration of an inert gas such as a nitrogen gas.

An extract may be concentrated and then used. A concentrated tea extract is obtained by concentrating an extract obtained by extraction with hot water or a water-soluble organic solvent from tea leaves, and prepared by the methods explained as examples in detail in JP-A-59-219384, JP-A-4-20589, JP-A-5-260907, JP-A-5-306279, and the like. Examples thereof include commercially available "Polyphenone" (Mitsui Norin Co., Ltd.), "TEAFURAN" (ITO EN LTD.), "Sunphenone" (Taiyo Kagaku Co., Ltd.), "Sunoolong" (Suntory Limited), and so forth. In addition, catechins derived from other raw materials, column-purified products, and chemically synthesized products can also be used as catechins. The concentrated tea extracts mentioned herein are in various forms such as a solid, an aqueous solution, and a slurry.

In the present invention, tea extracts and concentrated tea extracts can be used solely or in combination. Furthermore, a catechin can be ingested in the form of a liquid, a slurry, or a solid.

Polymerization of catechins proceeds in keeping with the ongoing fermentation of tea leaves before extraction proceeds. Therefore, when various concentrated tea extracts are added to water or a tea extract, concentrated green tea extracts are particularly preferred.

As the content of catechins in total polyphenols in a formulation, the percentage of non-epi-non-polymer catechins (A) and epi-non-polymer catechins (B) is preferably 50 to 100% by weight, more preferably 60 to 98% by weight of polyphenols, even more preferably 70 to 95% by weight. Here, the total polyphenol concentration means the quantity of polyphenols measured by the tartaric acid-iron method.

The weight ratio of non-epi-non-polymer catechins (A) to epi-non-polymer catechins (B) is preferably (B)/(A) = 0.5 to 20, more preferably 1 to 15, even more preferably 3 to 15. In this range, the improving effect of insulin sensitivity clearly manifests in obese children.

It is preferable that catechins selected from epigallocatechin gallate, gallocatechin gallate, epigallocatechin, and gallocatechin account for 30 to 98% by weight, preferably 40 to 90% by weight of the content of catechins because the flavor as a formulation becomes further superior, and the agent can be continuously used reasonably. Here, one or more of epigallocatechin gallate, gallocatechin gallate, epigallocatechin, and gallocatechin are contained.

To obtain the effect of the insulin sensitivity-improving agent of the present invention, the daily dose for children is 300 to 2500 mg as non-polymer catechins. In particular, the dose is preferably 400 to 1300 mg, more preferably 450 to 1300 mg, further preferably 500 to 800 mg.

When the present invention is utilized, it is easier in view of the quantity of catechins absorbed to obtain the insulin sensitivity improving effect of catechins when the daily dose of catechins is divided into a small number of times because the blood concentrations of catechins are increased.

Administration of the insulin sensitivity-improving agent of the present invention is targeted at obese children. The term "children" used herein means children (2 to 11 years of age) and adolescents (12 to 16 years of age). In addition, the term "obesity" in obese children means that the degree of obesity (=[actually measured body weight - standard body weight]/standard body weight x 100) is 20% or higher.

The insulin sensitivity-improving agent of the present invention is preferably intended for children aged 6 years or older, with the degree of obesity of 20 to 80%, preferably 30 to 80%, further preferably 30 to 50% in view of the effect thereof.

The present invention is intended for any obese children with decreased insulin sensitivity, who have a quantitative insulin sensitivity check index (QUICKI) of 0.31 or lower, even more preferably for those having the index of 0.21 to 0.30. To determine insulin sensitivity, the steady state plasma glucose (SSPG) method is used, in which endogenous insulin secretion is inhibited with somatostatin, predetermined amounts of insulin and glucose are intravenously and continuously infused, the blood sugar level that becomes virtually in equilibrium (steady-state plasma glucose) after the blood sugar level increases is used as an indicator of insulin sensitivity. However, since this method requires much time, equipment is also required, and further patients suffer from pain, usually a quantitative insulin sensitivity check index (QUICKI) attained by calculating 1/log(gluc*ins) from a fasting blood sugar level (gluc, mg/dL) and an insulin level (ins, mU/L) is used as the most convenient and common indicator.

It is sufficient that the intended children of the present invention are obese children with decreased insulin sensitivity. Decreased insulin sensitivity also causes insulin resistance. Therefore, the present invention is preferably intended for obese children with a value of the homeostasis model assessment of insulin resistance (HOMA-R), an indicator for insulin resistance, of 2 or higher, preferably 4 or higher, further preferably 5 or higher and 50 or lower. HOMA-R is calculated using the following equation.

$$\text{HOMA-R} = \text{fasting blood sugar (mg/dL)} \times \text{fasting blood insulin concentration (}\mu\text{U/mL)/405}$$

$$= \text{fasting blood sugar (mmol/L)} \times \text{fasting blood insulin concentration (}\mu\text{U/mL)/22.5}$$

The ingestion period for improving insulin sensitivity is preferably a period exceeding 8 weeks, particularly preferably 12 weeks or longer.

Examples of the formulations of the present invention include oral agents including solid formulations such as powders, granules, capsules, pills, and tablets, liquid solutions such as aqueous solutions, suspensions, and emulsions, and so forth. These oral agents are produced by adding excipients, disintegrating agents, binders, lubricants, surfactants, alcohols, water, water-soluble polymers, sweeteners, flavoring agents, acidifiers, and the like, which are commonly used depending on the form of an oral agent. Generally 0.1 to 100% by weight, particularly preferably 1 to 80% by weight of catechins are preferably contained in a drug for oral administration although it may vary depending on the purpose and the form of a drug.

When the present invention is used as a food, it can be used in various foods as a part of the food. The form of a food may be any of liquids or milky or pasty foods such as drinks, soy sauces, milks, yogurts, and fermented bean pastes, semisolid foods such as jellies and gummy sweets, solid foods such as gums, bean curds, and supplements, powder foods, and the like.

The insulin sensitivity-improving agent of the present invention is preferably in the form of a drink, in particular, in the form of a drink filled in a container because a catechin can be easily ingested, and the agent can be easily used continuously.

The content of a catechin in a drink is preferably 0.06 to 0.5% by weight, preferably 0.8 to 0.26% by weight, further preferably 0.10 to 0.26% by weight, even more preferably 0.12 to 0.16% by weight. This quantity is preferred because a large amount of the catechin can be easily ingested, while no intense bitter or astringent taste or no strong stypticity occurs. Furthermore, the content of 0.06% by weight or less is not preferred because the intake of a drink per day increases.

When the insulin sensitivity-improving agent of the present invention is produced in the form of a drink, non-epicatechins are preferably contained in a higher ratio because stability of the color tone is maintained over a long time. The weight ratio of non-epicatechins to epicatechins among catechins contained is 10:90 or higher, preferably 20:80 or higher, further preferably 30:70 or higher, most preferably 40:60 or higher in view of preservability as a drink.

Catechins in a drink are preferably in a dissolved state because it is easy to drink in view of flavor and smooth drinking, and is more likely to be continuously used.

To adjust the amount of catechins, a tea extract and a concentrated tea extract can be used in combination. Of the total contents of non-polymer catechins, the contents of catechins derived from concentrated tea extracts are 5 to 100%, further preferably 10 to 100%, even more preferably 20 to 100% in view of flavor.

The pH of a drink at 25°C is 3 to 7, preferably 4 to 7, even more preferably 5 to 7 in view of flavor and chemical stability of non-polymer catechins.

As a drink, by using other drinks such as fruit juices/fruit drinks, coffee drinks, oolong tea drinks, green tea drinks, black tea drinks, wheat tea drinks, and vegetable drinks in combination, a wide range of non-polymer catechin-containing drinks can be provided. For example, non-polymer catechins can be suitably added to soft drinks such as carbonated drinks, fruit-extract-containing drinks, vegetable-extract-containing juices, near water, sport drinks, and diet drinks.

Of these, a beverage which requires no sweetener (e.g. green teas, oolong teas and black teas) for its formulation is preferred. Furthermore, when a sweetener is used in black tea or the like, a low-calorie artificial sweetener is preferably used.

To the drink, additives such as antioxidants, flavors, various esters, organic acid, organic acid salts, inorganic acids, inorganic acid salts, inorganic salts, dyes, emulsifiers, preservatives, seasonings, sweeteners, acidulants, fruit juice extracts, vegetable extracts, nectar extracts, pH adjuster, and quality stabilizers may be used solely or in combination therewith, together with non-polymer catechins.

When a drink is produced as a drink filled in a container, the container used can be provided in usual forms such as molded containers (so-called PET bottle) containing polyethylene terephthalate as the principal component, metal can, paper containers combined with a metal foil or a plastic film, and bottles as in the case of common drinks.

Furthermore, the drink filled in a container is completely filled in a container, for example, as in a metal can, or degassing, nitrogen substitution, or both thereof are performed, and then, when heat sterilization can be performed, a drink filled in a container is produced under the sterilization conditions specified in the Food Sanitation Law. When retort sterilization cannot be performed as in the case of PET bottles and paper containers, methods are adopted in which the container is sterilized beforehand using, for example, a plate heat exchanger or the like at high temperature for a short period under sterilization conditions comparable to the above-mentioned conditions, then cooled to predetermined temperature, the drink is filled in the container, and the like. Other components may be added and filled in a filled container aseptically. A procedure can be performed in which pH is aseptically returned to neutral after heat sterilization under an acidic condition, pH is aseptically returned to an acidic condition after heat sterilization under a neutral condition, or the like.

Examples

Measurement of catechins

High performance liquid chromatography was performed by a gradient method at column temperature of 35°C by loading a drink filtered through a membrane filter (0.8 $\mu$m) on a high performance liquid chromatograph (Model No. SCL-10AVP) produced by Shimadzu Corporation using a packed column for octadecyl group introducing liquid chromatograph L-Column TM ODS (4.6 mm$\varphi$ x 250 mm: produced by Chemicals Evaluation and Research Institute, Japan). Mobile-phase solution A was a distilled aqueous solution containing 0.1 mol/L of acetic acid, and solution B was an acetonitrile solution containing 0.1 mol/L acetic acid. 20 $\mu$L of a sample was injected, and the UV detector wavelength was set at 280 nm.

Example 1

A double-blind parallel group comparison study was conducted, in which obese pediatric outpatients were assigned to receive a tea catechin-rich drink (576 mg of tea catechins was ingested daily, catechin group) or a control drink (75 mg of tea catechins, control group). The observation period before the study was set as 4 weeks, the study drink ingestion period as 24 weeks, and the observation period after the study as 12 weeks.

The insulin sensitivity-improving effect was evaluated before and after 24 weeks of ingestion of the study drink using the quantitative insulin sensitivity check index (QUICKI) attained by calculating 1/log(gluc*ins) from the fasting blood sugar level (gluc, mg/dL) and the insulin level (ins, mU/L).

The normal lower limit of QUICKI is defined as 0.357 to 0.382.

The changes in QUICKI at 24 weeks are shown in Tables 1 and 2 and Figure 1.
[Table 1]

| | Sex | Age | Degree of obesity | QUICKI | | | |
|---|---|---|---|---|---|---|---|
| | | | Initial value | QUICKI<0.310 | | QUICKI≥0.310 | |
| | | | | Initial value | Change at 24 weeks | Initial value | Change at 24 weeks |
| Catechin group | Male | 11 | 32.8 | 0.215 | 0.043 | | |
| Catechin group | Female | 15 | 28.2 | 0.275 | -0.004 | | |
| Catechin group | Female | 14 | 80.0 | 0.283 | 0.001 | | |
| Catechin group | Male | 9 | 42.3 | 0.286 | -0.017 | | |
| Catechin group | Female | 11 | 39.7 | 0.292 | 0.010 | | |
| Catechin group | Female | 12 | 56.8 | 0.296 | -0.025 | | |
| Catechin group | Female | 13 | 49.8 | 0.299 | 0.003 | | |
| Catechin group | Female | 13 | 25.2 | 0.304 | 0.006 | | |
| Catechin group | Male | 11 | 32.4 | 0.305 | -0.024 | | |
| Catechin group | Female | 10 | 33.9 | 0.306 | 0.018 | | |
| Catechin group | Female | 12 | 33.5 | 0.308 | 0.011 | | |
| Catechin group | Female | 11 | 40.4 | | | 0.311 | -0.015 |
| Catechin group | Female | 10 | 44.2 | | | 0.318 | -0.007 |
| Catechin group | Female | 11 | 28.1 | | | 0.321 | -0.015 |
| Catechin group | Male | 6 | 33.5 | | | 0.323 | -0.038 |
| Catechin group | Female | 10 | 68.9 | | | 0.325 | -0.020 |
| Catechin group | Male | 14 | 31.6 | | | 0.327 | 0.030 |
| Control group | Female | 11 | 43.4 | 0.275 | 0.016 | | |
| Control group | Female | 12 | 34.1 | 0.279 | 0.021 | | |
| Control group | Male | 12 | 51.3 | 0.286 | 0.035 | | |
| Control group | Female | 10 | 52.0 | 0.286 | 0.034 | | |

(continued)

| | Sex | Age | Degree of obesity | QUICKI | | | |
|---|---|---|---|---|---|---|---|
| | | | Initial value | QUICKI<0.310 | | QUICKI≥0.310 | |
| | | | | Initial value | Change at 24 weeks | Initial value | Change at 24 weeks |
| Control group | Female | 14 | 91.7 | 0.286 | -0.049 | | |
| Control group | Female | 13 | 63.5 | 0.287 | -0.016 | | |
| Control group | Female | 9 | 67.6 | 0.287 | -0.003 | | |
| Control group | Female | 9 | 62.7 | 0.296 | -0.015 | | |
| Control group | Female | 10 | 22.0 | 0.299 | -0.016 | | |
| Control group | Male | 16 | 57.5 | 0.307 | -0.002 | | |
| Control group | Female | 9 | 44.0 | | | 0.310 | -0.015 |
| Control group | Male | 12 | 27.2 | | | 0.310 | -0.030 |
| Control group | Male | 11 | 41.3 | | | 0.311 | 0.011 |
| Control group | Female | 16 | 42.6 | | | 0.312 | 0.017 |
| Control group | Female | 12 | 33.3 | | | 0.315 | -0.007 |
| Control group | Female | 6 | 37.0 | | | 0.316 | -0.030 |
| Control group | Female | 14 | 25.4 | | | 0.320 | 0.039 |
| Control group | Male | 14 | 17.0 | | | 0.321 | 0.031 |
| Control group | Male | 14 | 13.1 | | | 0.332 | -0.018 |
| Mean | Catechin group | 11.4 | 41.2 | 0.288 | 0.002 | 0.321 | -0.011 |
| | Control group | 11.8 | 43.5 | 0.289 | 0.001 | 0.316 | 0.000 |
| Standard deviation | Catechin group | 2.1 | 15.0 | 0.026 | 0.020 | 0.006 | 0.023 |
| | Control group | 2.6 | 19.6 | 0.009 | 0.026 | 0.007 | 0.026 |

[Table 2]

| QUICKI stratified using initial value of HOMA-R | | | | | | | | |
|---|---|---|---|---|---|---|---|---|
| | Sex | HOMA-R | QUICKI | | | | | |
| | | Initial value | HOMA-R<4 | | 4≤HOMA-R<5 | | 5≤HOMA-R<50 | |
| | | | Initial value | Change at 24 weeks | Initial value | Change at 24 weeks | Initial value | Change at 24 weeks |
| Catechin group | Male | 2.8 | 0.327 | 0.030 | | | | |
| Catechin group | Female | 2.9 | 0.325 | -0.020 | | | | |

(continued)

| | Sex | HOMA-R | QUICKI | | | | | |
| --- | --- | --- | --- | --- | --- | --- | --- | --- |
| QUICKI stratified using initial value of HOMA-R | | | | | | | | |
| | | Initial value | HOMA-R<4 | | 4≤HOMA-R<5 | | 5≤HOMA-R<50 | |
| | | | Initial value | Change at 24 weeks | Initial value | Change at 24 weeks | Initial value | Change at 24 weeks |
| Catechin group | Male | 3.1 | 0.323 | -0.038 | | | | |
| Catechin group | Female | 3.2 | 0.321 | -0.015 | | | | |
| Catechin group | Female | 3.5 | 0.318 | -0.007 | | | | |
| Catechin group | Female | 4.0 | | | 0.311 | -0.015 | | |
| Catechin group | Female | 4.3 | | | 0.308 | 0.011 | | |
| Catechin group | Female | 4.6 | | | 0.306 | 0.018 | | |
| Catechin group | Male | 4.7 | | | 0.305 | -0.024 | | |
| Catechin group | Female | 4.8 | | | 0.304 | 0.006 | | |
| Catechin group | Female | 5.5 | | | | | 0.299 | 0.003 |
| Catechin group | Female | 5.8 | | | | | 0.296 | -0.025 |
| Catechin group | Female | 6.6 | | | | | 0.292 | 0.010 |
| Catechin group | Male | 7.6 | | | | | 0.286 | -0.017 |
| Catechin group | Female | 8.4 | | | | | 0.283 | 0.001 |
| Catechin group | Female | 10.5 | | | | | 0.275 | -0.004 |
| Catechin group | Female | 17.9 | | | | | 0.259 | 0.044 |
| Control group | Male | 2.5 | 0.332 | -0.018 | | | | |
| Control group | Male | 3.2 | 0.321 | 0.031 | | | | |
| Control group | Female | 3.3 | 0.320 | 0.039 | | | | |
| Control group | Female | 3.6 | 0.316 | -0.030 | | | | |
| Control group | Female | 3.7 | 0.315 | -0.007 | | | | |

(continued)

| QUICKI stratified using initial value of HOMA-R | | | | | | | | | |
|---|---|---|---|---|---|---|---|---|---|
| | Sex | HOMA-R | QUICKI | | | | | | |
| | | Initial value | HOMA-R<4 | | 4≤HOMA-R<5 | | 5≤HOMA-R<50 | | |
| | | | Initial value | Change at 24 weeks | Initial value | Change at 24 weeks | Initial value | Change at 24 weeks | |
| Control group | Female | 4.0 | | | 0.312 | 0.017 | | |
| Control group | Male | 4.0 | | | 0.311 | 0.011 | | |
| Control group | Male | 4.1 | | | 0.310 | -0.030 | | |
| Control group | Female | 4.2 | | | 0.310 | -0.015 | | |
| Control group | Male | 4.4 | | | 0.307 | -0.002 | | |
| Control group | Female | 5.5 | | | | | 0.299 | -0.016 |
| Control group | Female | 5.8 | | | | | 0.296 | -0.015 |
| Control group | Female | 7.5 | | | | | 0.287 | -0.003 |
| Control group | Female | 7.6 | | | | | 0.287 | -0.016 |
| Control group | Female | 7.6 | | | | | 0.286 | -0.049 |
| Control group | Female | 7.7 | | | | | 0.286 | 0.034 |
| Control group | Male | 7.8 | | | | | 0.286 | 0.035 |
| Control group | Female | 9.6 | | | | | 0.279 | 0.021 |
| Control group | Female | 10.5 | | | | | 0.275 | 0.016 |
| Mean | Catechin group | 11.8 | 0.323 | -0.010 | 0.307 | -0.001 | 0.284 | 0.002 |
| | Control group | 5.6 | 0.321 | 0.003 | 0.310 | -0.004 | 0.287 | 0.001 |
| Standard deviation | Catechin group | 25.2 | 0.004 | 0.025 | 0.002 | 0.019 | 0.007 | 0.022 |
| | Control group | 2.4 | - | - | 0.002 | 0.019 | 0.007 | 0.028 |

The X-axis and the Y-axis in Figure 1 can be defined as follows.

X-axis: represents the quantitative insulin sensitivity check index (QUICKI) before ingestion of the drink. The lower the value, the worse the insulin sensitivity is. Y-axis: represents a difference obtained by deducting QUICKI after 24 weeks of continuous use of the drink from the initial QUICKI. (-) denotes the worsening of insulin sensitivity, and (+) denotes the improvement of insulin sensitivity.

In the catechin group, significant negative correlations were observed between the initial QUICKI values and the changes at 24 weeks. This demonstrates that continuous use of the catechin drink leads to improvement in obese children with poor insulin sensitivity and exhibits no effect on obese children with favorable insulin sensitivity. On the other hand, no significant relationship was observed between the initial QUICKI values and the changes at 24 weeks in the control group, demonstrating that continuous use of the control drink (corresponding to a common tea) had no effect on QUICKI.

As shown in Table 1 and Figure 1, there is significantly a negative correlation between the initial values and the changes at 24 weeks in the catechin group. The catechin group also shows that the worse the insulin sensitivity of a patient was, the more its improvement is made possible. In contrast, significant correlations were not observed in the control group. Furthermore, as shown in Table 2, the effects of catechins were marked in obese children with HOMA-R of 5 or higher.

## Claims

1. A catechin for use in a method for improving insulin sensitivity in an obese child with a quantitative insulin sensitivity check index (QUICKI) of 0.31 or lower.

2. The catechin for use as claimed in claim 1, wherein the catechin is a non-polymer catechin.

3. The catechin for use as claimed in any one of claims 1 or 2, wherein the obese child has an indicator for insulin resistance (HOMA-R) of 5 or higher.

4. The catechin for use as claimed in any one of claims 1 to 3, wherein the obese child is 6 to 16 years of age.

5. The catechin for use as claimed in any one of claims 1 to 4, wherein the insulin sensitivity-improving agent is in the form of a drink.

## Patentansprüche

1. Ein Catechin zur Verwendung in einem Verfahren zum Verbessern der Insulinsensitivität in einem fettleibigen Kind mit einem quantitativen Insulinsensitivitätscheckindex (QUICKI) von 0,31 oder niedriger.

2. Catechin zur Verwendung gemäß Anspruch 1, worin das Catechin ein nicht-polymeres Catechin ist.

3. Catechin zur Verwendung gemäß irgendeinem der Ansprüche 1 oder 2, worin das fettleibige Kind einen Indikator für Insulinresistenz (HOMA-R) von 5 oder höher hat.

4. Catechin zur Verwendung gemäß irgendeinem der Ansprüche 1 bis 3, worin das fettleibige Kind 6 bis 16 Jahre alt ist.

5. Catechin zur Verwendung gemäß irgendeinem der Ansprüche 1 bis 4, worin das insulinsensitivitätsverbessernde Mittel in Form eines Getränks vorliegt.

## Revendications

1. Catéchine pour une utilisation dans un procédé d'amélioration de la sensibilité à l'insuline chez un enfant obèse avec un indice quantitatif de contrôle de la sensibilité à insuline (QUICKI) de 0,31 ou moins.

2. Catéchine pour une utilisation telle que revendiquée selon la revendication 1, dans laquelle la catéchine est une catéchine non-polymère.

3. Catéchine pour une utilisation telle que revendiquée selon l'une quelconque des revendications 1 ou 2, dans laquelle l'enfant obèse a un indicateur de résistance à l'insuline (HOMA-R) de 5 ou plus.

**4.** Catéchine pour une utilisation telle que revendiquée selon l'une quelconque des revendications 1 à 3, dans laquelle l'enfant obèse est âgé de 6 à 16 ans.

**5.** Catéchine pour une utilisation telle que revendiquée selon l'une quelconque des revendications 1 à 4, dans laquelle l'agent améliorant la sensibilité à l'insuline est sous la forme d'une boisson.

Fig. 1

CHANGES IN QUICKI AT 24 WEEKS

AGGRAVATED←INSULIN SENSITIVITY→IMPROVED

0.06
0.04
0.02
0.00
-0.02
-0.04
-0.06

0.25    0.30    0.35

POOR←INSULIN SENSITIVITY→FAVORABLE
INITIAL QUICKI VALUE

●CATECHIN GROUP
○CONTROL GROUP

●CATECHIN GROUP
CHANGES IN QUICKI AT 24 WEEKS = 0.127 – 0.429 · INITIAL QUICKI VALUE; $R^2$ = 0.146
○CONTROL GROUP
CHANGES IN QUICKI AT 24 WEEKS = 0.042 – 0.14 · INITIAL QUICKI VALUE; $R^2$ = 0.008

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 60156614 A
- JP 3133928 A
- JP 59219384 A
- JP 4020589 A
- JP 5260907 A
- JP 5306279 A

### Non-patent literature cited in the description

- *Biochemistry,* 1999, vol. 71 (11), 1281-1298
- *Chemistry and Biology,* vol. 37 (2), 120-125
- *Laboratory Tests,* 1998, vol. 42 (4), 395-403
- Multiple risk factor syndrome. 1999, 2
- **Fukino Yoko et al.** *J. Nutrit. Sci. Vit.,* October 2005, vol. 51 (5), 335-342
- **Ryu O H et al.** *Diabetes Res. Clin. Pract.,* 01 March 2006, vol. 71 (3), 356-358